# FASCICULE DE BREVET EUROPEEN

(11) **EP 2 670 450 B1**
(45) Date de publication et mention de la délivrance du brevet: **03.05.2017**
(21) Numéro de dépôt: 12707826.9
(22) Date de dépôt: 31.01.2012
(51) Int. Cl.: A61M 1/00

(54) **MOUCHE-BEBE**
NASALASPIRATOR FÜR BABYS
NASAL ASPIRATOR FOR BABIES

(30) Priorité: 02.02.2011 FR 1150796
(43) Date de publication de la demande: 11.12.2013
(73) Titulaire: Lambert, Cyril, 33000 Bordeaux (FR); Kinkingnehun, Serge, 94400 Vitry-sur-Seine (FR); Vasseur, Franz, 75011 Paris (FR)
(72) Inventeur: LAMBERT, Cyril, 33000 Bordeaux (FR)
(74) Mandataire: Aquinov
(86) Numéro de dépôt international: PCT/FR2012/050203
(87) Numéro de publication internationale: WO 2012/104540

(56) Documents cités:
- FR-A1- 2 549 727
- US-A- 5 183 467

## Description

La présente invention se rapporte à un dispositif permettant d'aspirer les sécrétions nasales communément appelé mouche-bébé.

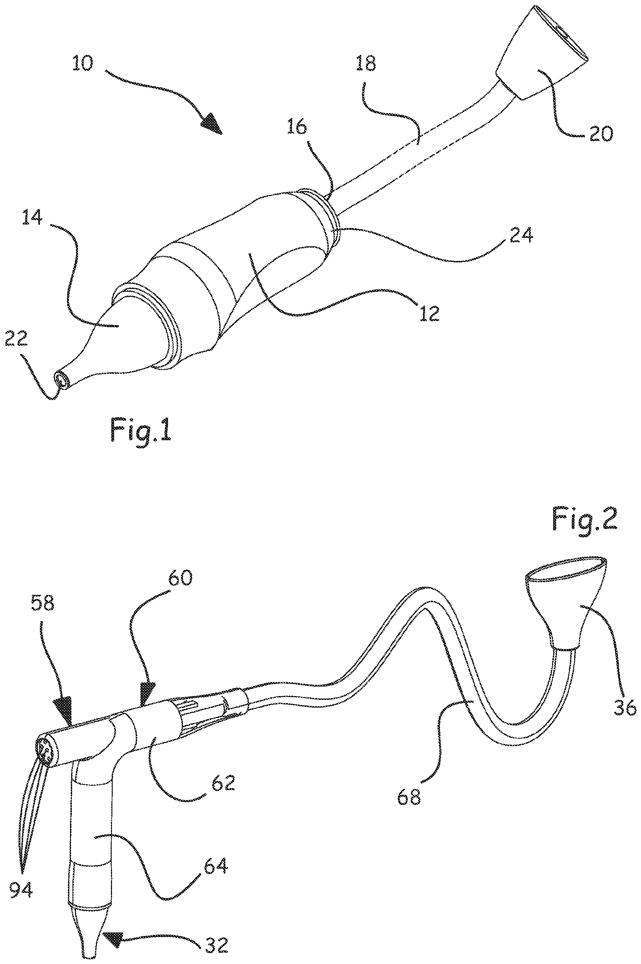

Comme illustré sur la figure 1, un mouche-bébé 10 de l'art antérieur comprend un corps creux 12 avec à une première extrémité un embout nasal 14 et à l'autre extrémité un orifice 16, un tube souple 18 reliant l'orifice 16 à un embout de bouche 20. Un autre dispositif est décrit dans document US5183467. L'embout nasal 14 comprend à une extrémité un diamètre extérieur réduit de manière à être introduit dans les narines d'un nourrisson. Cette même extrémité comprend également un orifice 22 via lequel sont aspirées les sécrétions nasales. Selon un mode de réalisation, le corps creux 12 se présente sous la forme d'un cylindre creux, l'embout nasal 14 étant emmanché à une première extrémité, un bouchon 24 en forme de demi sphère avec l'orifice 16 étant emmanché à l'autre extrémité.

Le fonctionnement d'un mouche-bébé est relativement simple. L'extrémité de l'embout nasal 14 est introduit dans l'une des narines du nourrisson et l'embout de bouche 20 est mis en bouche par la personne chargée d'extraire les sécrétions nasales.

Cette dernière en aspirant provoque l'extraction des sécrétions de la narine qui passent via l'embout nasal dans le corps creux 12.

Pour limiter le transfert des sécrétions jusqu'à la bouche de la personne qui aspire, le corps comprend un filtre sous forme d'un bouchon en mousse dont le diamètre extérieur est adapté au diamètre intérieur du cylindre creux formant le corps creux 12.

Cette solution simple n'est pas satisfaisante car ce filtre ne permet pas de retenir les éléments de faibles dimensions tels que les virus qui migrent de la narine du nourrisson jusqu'à la bouche de la personne qui aspire et la contaminent.

Pour pallier cet inconvénient, certains mouche-bébés comprennent des moyens d'aspiration motorisés. Même si cette solution permet de limiter les risques de contamination de la personne qui utilise le mouche-bébé dans la mesure-où elle n'a plus besoin d'aspirer, elle n'est pas pleinement satisfaisante car elle conduit à augmenter de manière significative le coût de revient du mouche-bébé.

Selon l'art antérieur, on connait différents appareils pour aspirer des sécrétions qui sont décrits par exemple dans les documents US-5.183.476, US-3.833.001, FR-2.549.727. Selon ces documents, on utilise pour provoquer l'aspiration, l'écoulement d'un fluide qui grâce à un effet venturi entraîne un flux d'air qui engendre l'aspiration. Selon ces documents, le fluide utilisé est de l'eau et le flux de l'eau provient d'un robinet ou d'une pompe. D'autres documents proposent d'utiliser comme fluide d'entrainement de l'air. Toutefois, comme précédemment, le flux d'air est provoqué par une pompe. Compte tenu des moyens utilisés pour provoquer l'effet venturi, ces dispositifs nécessitent une pompe ou tout autre mécanisme pour produire le flux d'air apte à générer un flux d'aspiration grâce à un effet venturi.

Aussi, la présente invention vise à pallier les inconvénients de l'art antérieur en proposant un mouche-bébé de conception simple afin d'en limiter les coûts, tout en réduisant les risques de contamination de la personne qui l'utilise.

A cet effet, l'invention a pour objet un dispositif permettant d'aspirer les sécrétions nasales comprenant un embout nasal avec un orifice via lequel sont aspirées les sécrétions nasales, un premier conduit dont une première extrémité est reliée à l'embout nasal, l'autre extrémité étant débouchante via un premier orifice et un second conduit avec une extrémité débouchante via un second orifice, caractérisé en ce que les deux conduits forment un angle inférieur ou égal à 90° et l'orifice relié à l'embout nasal est disposé dans le prolongement du second conduit et dans la veine d'air sortant de l'orifice du second conduit de manière à provoquer un effet venturi et un phénomène d'aspiration dans le premier conduit relié à l'embout nasal.

D'autres caractéristiques et avantages ressortiront de la description qui va suivre de l'invention, description donnée à titre d'exemple uniquement, en regard des dessins annexés sur lesquels :
- La figure 1 est une vue en perspective d'un mouche-bébé selon l'art antérieur,
- La figure 2 est une vue en perspective d'un mouche-bébé selon l'invention,
- La figure 3 est une vue en coupe du mouche-bébé de la figure 2,
- La figure 4 est une vue en coupe des différents éléments du mouche-bébé de la figure 2,
- La figure 5 est une vue en perspective d'une variante de l'invention,
- La figure 6 est une vue en perspective d'un mouche-bébé selon une autre variante de l'invention,
- La figure 7 est une vue en perspective illustrant le mouche-bébé de la figure 6 en position ouverte, et
- La figure 8 est une coupe du mouche-bébé de la figure 6.

De manière connue, un mouche-bébé 30 comprend d'une part, un embout nasal 32 avec, à une extrémité susceptible d'être introduite dans la narine d'un nourrisson, un orifice 34 via lequel sont aspirées les sécrétions nasales, et d'autre part, un embout de bouche 36 susceptible d'être mis au moins partiellement dans la bouche d'une personne utilisant le mouche-bébé 30.

Selon l'invention, le mouche-bébé 30 comprend d'une part un premier conduit 38 dont une première extrémité est reliée à l'embout nasal 32, l'autre extrémité étant débouchante via un orifice 40, et d'autre part, un second conduit 42 dont une première extrémité est reliée à l'embout de bouche 36, l'autre extrémité étant débouchante via un orifice 44. Les deux conduits 38 et 42 et les deux orifices 40 et 44 sont disposés de manière à ce qu'un flux d'air sortant de l'orifice 44 relié à l'embout de bouche 36 provoque un effet venturi et un phénomène d'aspiration dans le premier conduit 38 relié à l'embout nasal 32.

A cet effet, les deux conduits 38, 42 forment un angle inférieur ou égal à 90° et l'orifice 40 relié à l'embout nasal est disposé dans le prolongement du second conduit 42, dans la veine d'air sortant de l'orifice 44 relié à l'embout de bouche 36. De préférence, les deux conduits 38, 42 sont disposés approximativement à 90°.

Cet agencement des conduits permet de pouvoir générer une aspiration satisfaisante en soufflant dans l'embout de bouche 36.

Selon un mode de réalisation, les deux conduits 38 et 42 se présentent chacun sous la forme d'un cylindre creux respectivement d'axes 46 et 48. L'orifice 40 est délimité par un bord périphérique disposé dans un plan perpendiculaire à l'axe 46. L'orifice 44 est délimité par un bord périphérique disposé dans un plan perpendiculaire à l'axe 48. De préférence, le bord périphérique délimitant l'orifice 40 relié à l'embout nasal 32 est disposé dans un plan contenant l'axe du conduit 42 relié à l'embout de bouche 36. Cet agencement permet d'améliorer l'effet Venturi.

Avantageusement, la section du conduit 38 au niveau de l'orifice 40 est inférieure à celle du conduit 42 au niveau de l'orifice 44. De préférence, le diamètre du conduit 38 au niveau de l'orifice 40 est égal à approximativement la moitié du diamètre du conduit 42 au niveau de l'orifice 44. Cet agencement permet d'améliorer l'effet Venturi.

Contrairement à l'art antérieur, l'utilisateur n'aspire plus au niveau de l'embout de bouche pour provoquer l'aspiration des sécrétions nasales au niveau de l'embout nasal. Il lui suffit de souffler dans l'embout de bouche pour provoquer l'aspiration des sécrétions nasales au niveau de l'embout nasal ce qui limite les risques de contamination lors de l'utilisation.

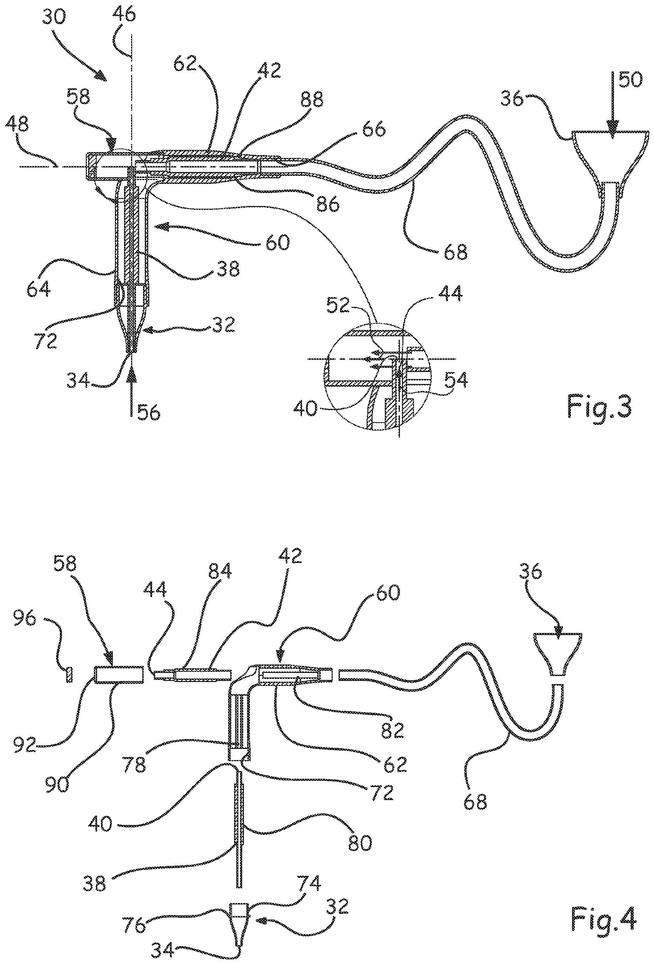

Comme illustré sur la figure 3, lorsque l'utilisateur souffle dans l'embout de bouche (comme illustré par la flèche 50), un flux d'air sort du conduit 42 via l'orifice 44 (comme illustré par les flèches 52). Ce flux d'air sortant du conduit 42 provoque un effet venturi au niveau du conduit 38 qui génère un flux sortant dudit conduit 38 (matérialisé par la flèche 54) et une aspiration au niveau de l'embout nasal (matérialisée par la flèche 56).

Avantageusement, le mouche-bébé 30 comprend un réservoir 58 permettant de collecter les sécrétions nasales aspirées dans lequel découchent les conduits 38 et 42 via les orifices 40 et 44.

De préférence, les conduits 38 et 42 sont rigides au moins au niveau des orifices 40 et 44.

Selon un mode de réalisation préféré et illustré sur les figures 2 à 4, le mouche-bébé 30 comprend un corps rigide 60 en forme de L, avec une première branche 62 creuse dans laquelle se loge le conduit 42 et une seconde branche 64 creuse dans laquelle se loge le conduit 38.

La branche 62 se présente sous la forme d'un tube avec des sections circulaires et comprend à l'extrémité non reliée à la branche 64 un orifice 66 permettant le passage du second conduit 42 et/ou d'un tube souple 68 reliant ledit conduit 42 à l'embout de bouche 36. L'autre extrémité du conduit 62 est débouchante de manière à recevoir par emboitement le réservoir 58.

La branche 64 se présente sous la forme d'un tube avec des sections circulaires et comprend à l'extrémité non reliée à la branche 62 un orifice 72 permettant d'emmancher un embout nasal 32.

Selon un mode de réalisation, l'embout nasal 32 comprend une forme tronconique avec à une extrémité l'orifice 34 et à l'autre extrémité une portée 74 limitée par une collerette 76, ladite portée étant susceptible de s'emmancher dans la branche 64 jusqu'à la collerette 76. L'embout nasal 32 n'est pas plus décrit car il peut être identique à celui de l'art antérieur.

Les conduits 38 et 42 sont positionnés dans le corps rigide 60 de manière à ce que les orifices 40 et 42 soient correctement positionnés.

Selon un mode de réalisation, la branche 64 et/ou le conduit 38 comprennent des moyens pour positionner le conduit 38 dans un plan perpendiculaire à l'axe 46. En complément, la branche 64 et/ou l'embout nasal 32 comprennent des moyens pour positionner le conduit 38 selon la direction de l'axe 46 de manière à ce que l'orifice 40 du conduit 38 soit correctement positionné par rapport à l'orifice 44 du conduit 42.

A cet effet, la branche 64 comprend au niveau de sa paroi intérieure des nervures 78 permettant de centrer le conduit 38. En parallèle, le conduit 38 comprend au niveau de sa paroi extérieure des nervures 80 permettant de le centrer dans la branche 64. En complément, une extrémité du conduit 38 vient s'emmancher dans l'orifice 34 de l'embout nasal pour le positionner selon la direction de l'axe 46.

En parallèle, la branche 62 et/ou le conduit 42 comprennent des moyens pour positionner le conduit 42 dans un plan perpendiculaire à l'axe 48 et selon la direction de l'axe 48 de manière à ce que l'orifice 44 du conduit 42 soit correctement positionné par rapport à l'orifice 40 du conduit 38.

A cet effet, la branche 62 comprend au niveau de sa paroi intérieure des nervures 82 permettant de centrer le conduit 42. En parallèle, le conduit 42 comprend au niveau de sa paroi extérieur des nervures 84 pour se centrer dans la branche 62.

Pour positionner le conduit 42 selon l'axe 48, ce dernier comprend une réduction de son diamètre extérieur qui forme un épaulement 86 qui vient en appui contre une paroi 88 prévue à l'intérieur de la branche 62.

En complément, une extrémité du conduit 42 vient s'emmancher dans l'orifice 66 de la branche 62 pour positionner le conduit 42 selon la direction de l'axe 48. Le tube souple 68 vient s'emmancher dans le conduit 42 au niveau de l'orifice 66.

Selon un mode de réalisation, le réservoir 58 se présente sous la forme d'un cylindre 90 fermé à une extrémité par une paroi 92 avec des orifices 94 permettant le passage de l'air et dont le diamètre extérieur est ajusté au diamètre intérieur du conduit formant la branche 62 de manière à s'emmancher dans ledit conduit.

La paroi cylindrique 90 du réservoir comprend une découpe en U de manière à permettre le passage du conduit 38.

Un filtre 96 sous forme d'une pastille en mousse est avantageusement prévu à l'intérieur du réservoir 58, plaqué contre la paroi 92.

Selon un mode de réalisation, les éléments constituant le mouche-bébé sont réalisés en matière plastique. Les liaisons entre ces différents éléments sont réalisés par emmanchement ou par emboitement de manière à simplifier le montage et le démontage et permettre un nettoyage aisé.

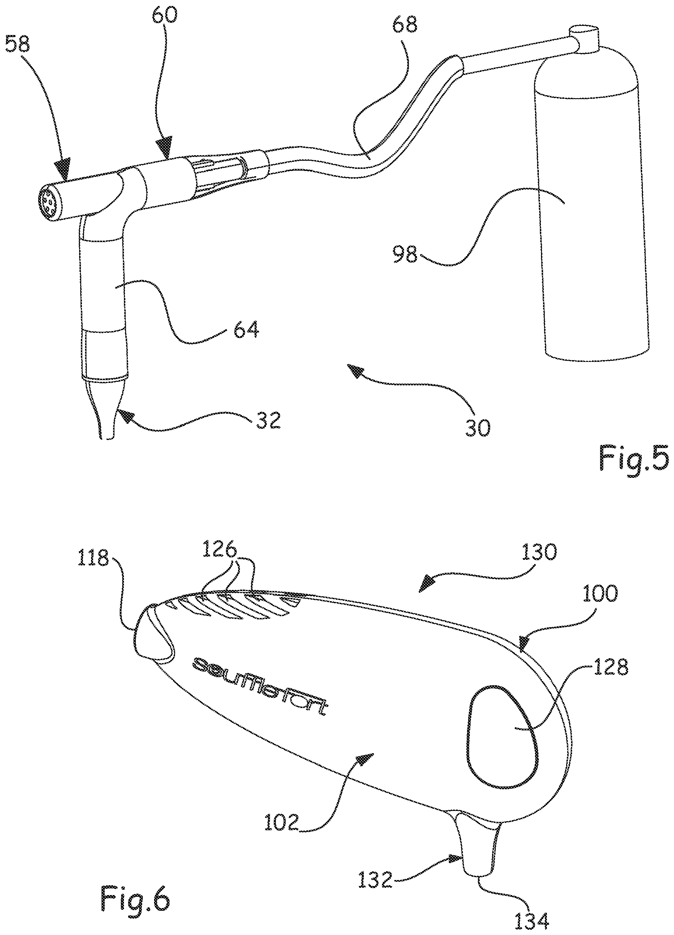

Selon une autre variante illustrée sur la figure 5, le mouche-bébé 30 peut ne pas comprendre d'embout de bouche 36. Pour générer le flux d'air sortant du second conduit 42 via l'orifice 44, on peut relier l'autre extrémité du conduit 42 à un réservoir 98 de gaz sous pression. Ce réservoir 98 peut se présenter sous la forme d'un aérosol dont la valve de sortie est reliée au conduit 42 directement ou par l'intermédiaire d'un tube souple analogue au tube souple 68. Ce réservoir peut contenir de l'air sec sous pression.

Selon un autre mode de réalisation décrit sur les figures 6 à 8, un mouche-bébé 130 comprend comme précédemment deux conduits 138 et 142 avec respectivement des axes 146 et 148, le premier étant relié à un embout nasal 132, le second à un embout de bouche 136 et dont les orifices respectifs 140 et 144 sont agencés conformément à l'invention. Selon ce mode de réalisation, le mouche-bébé 130 comprend deux demi-coques 100 et 102 qui sont assemblées selon un plan de jonction P contenant les axes 146 et 148 et qui ont des parois 104 et 106 sensiblement symétriques par rapport au plan P. Les deux parois 104 et 106 ont chacune une surface extérieure qui constitue la surface extérieure du mouche-bébé. Lorsque les deux demi-coques sont assemblées le mouche-bébé a une forme ovoïde allongée. La dimension du mouche-bébé selon la direction de l'axe 148 est au moins deux fois supérieure à la dimension selon la direction de l'axe 146. Cette forme ovoïde allongée est sensiblement aplatie selon la direction perpendiculaire au plan P.

Cette forme ovoïde est sensiblement symétrique par rapport à un plan perpendiculaire au plan P et passant par l'axe 148.

Pour donner un ordre de grandeur et de manière non limitative, la forme ovoïde a une longueur de l'ordre de 12 cm selon la direction de l'axe 148, une largeur de l'ordre de 5 cm selon la direction de l'axe 146.

La forme ovoïde comprend un appendice qui forme l'embout nasal 132. Cette appendice a une forme tronconique avec une surface plane 108 perpendiculaire au plan P et parallèle à l'axe 148, contenant l'orifice 134 de l'embout nasal. Avantageusement, l'embout nasal 132 est constitué de deux parties symétriques par rapport au plan P. Ces deux parties sont intégrées aux deux demi-coques 100 et 102 et sont obtenues d'un seul tenant avec ces dernières.

De préférence, l'embout nasal 132 comprend un revêtement 110 hypoallergénique au niveau de sa surface extérieure.

Selon un mode de réalisation préféré, le conduit 138 s'étend depuis la face plane 108 jusqu'à l'axe 148. Il a une longueur de l'ordre de 4 cm, un diamètre intérieur de l'ordre de 4,5 mm et un diamètre intérieur de l'ordre de 3,5 mm.

Le conduit 142 est disposé à l'intérieur de la forme ovoïde. Il a une longueur de l'ordre de 7,7 cm et son extrémité orientée vers le premier conduit 138 vient de préférence en contact avec ce dernier.

Selon une caractéristique de l'invention, le second conduit 142 a un diamètre au niveau de son orifice 144 inférieur au diamètre prévu à l'autre extrémité reliée à l'embout de bouche.

Ainsi, le second conduit a un diamètre intérieur de l'ordre de 4,5 mm au niveau de l'orifice 144 et un diamètre intérieur de l'ordre de 5,5 mm à l'autre extrémité. Cette réduction du diamètre permet d'augmenter la vitesse d'écoulement du flux d'air à l'intérieur du second tube 142 ce qui contribue à améliorer l'effet d'aspiration.

Selon un mode de réalisation préféré, les deux conduits 138 et 142 sont solidaires d'une demi-coque 100 et sont réalisés d'un seul tenant avec cette demi-coque 100. En complément, l'autre demi-coque 102 comprend des rigoles 112 dont les formes correspondent aux surfaces extérieures des conduits 138 et 142.

Pour assurer le positionnement relatif des deux demi-coques 100 et 102, les surfaces extérieures des deux conduits 138 et 142 comprennent des formes en saillie 114 qui coopèrent avec des formes en creux 116 au niveau des rigoles 112. Les deux demi-coques comprennent chacune dans le prolongement du second conduit 142 une empreinte demi-cylindrique 118 permettant d'emmancher un tube souple 120 qui relie le second conduit 142 à l'embout de bouche 136.

L'intérieur des deux demi-coques 100 et 102 forme un réservoir 122 permettant de collecter les sécrétions.

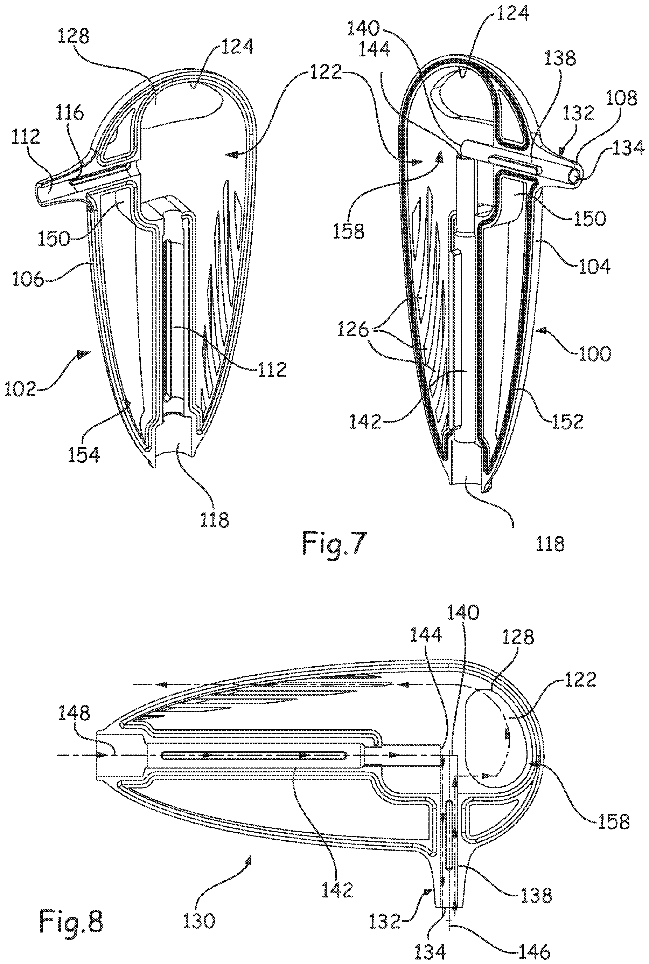

Le réservoir 122 a des formes permettant d'orienter le flux d'air sortant du second conduit 142 de manière à ce qu'il fasse un demi-tour comme illustré sur la figure 8. Cet agencement permet de séparer les sécrétions du flux d'air.

A cet effet, le réservoir a un profil courbe 124 en face du conduit 142 de manière à imprimer au flux d'air un demi-tour.

Pour assurer la sortie du flux d'air à l'extérieur du mouche-bébé 130, au moins une sortie d'air 126 est prévue sur au moins une demi-coque. Avantageusement, les deux demi-coques 100 et 102 comprennent des sorties d'air 126 réparties de manière sensiblement symétrique par rapport au plan P.

Pour renforcer l'effet procurer par le profil courbe 124, les sorties d'air 126 sont disposées à l'opposé du profil courbe 124 dans une zone du réservoir décalée par rapport à l'extrémité 144 du conduit 142 en direction de l'autre extrémité du conduit 142.

Pour visualiser les sécrétions, au moins une demi-coque 100 ou 102 comprend au niveau de la paroi 104 une portion 128 transparente. De préférence, chaque demi-coque 100 et 102 comprend une portion transparente 128.

De préférence, l'intérieur des deux demi-coques 100 et 102 comprend au moins une cloison 150 permettant de réduire le volume du réservoir afin de limiter la zone à nettoyer. Ainsi, le réservoir 122 est délimité par le second conduit 142 et une cloison 150 sécante avec le premier conduit 138 et qui s'étend jusqu'au profil courbe 124. Selon un mode de réalisation, la cloison 150 est constituée de deux parties, une pour chaque demi-coque 100 et 102, jointives au niveau du plan de jonction P.

Pour assurer l'étanchéité, le bord périphérique de la demi-coque 100 comprend au moins une nervure 152 qui se loge dans au moins une gorge 154 prévue au niveau du bord périphérique de la demi-coque 102.

Avantageusement, les nervures 152 s'étendent sur la périphérie de la demi-coque 100, de part et d'autre de chaque conduit 138 et 142 et au niveau de la cloison 150. Il en est de même des gorges 154.

De préférence, les nervures 152 ont une hauteur inférieure à celle des formes en saillie 114 qui assurent le positionnement des deux demi-coques 100 et 102.

## Revendications

1. Dispositif permettant d'aspirer les sécrétions nasales comprenant un embout nasal (32, 132) avec un orifice (34, 134) via lequel sont aspirées les sécrétions nasales, un premier conduit (38, 138) dont une première extrémité est reliée à l'embout nasal (32, 132), l'autre extrémité étant débouchante via un premier orifice (40, 140) et un second conduit (42, 142) avec une extrémité débouchante via un second orifice (44, 144), **caractérisé en ce que** les deux conduits (38, 42, 138, 142) forment un angle inférieur ou égal à 90° et l'orifice (40, 140) relié à l'embout nasal est disposé dans le prolongement du second conduit (42, 142), dans la veine d'air sortant de l'orifice (44, 144) du second conduit (42, 142).

2. Dispositif selon la revendication 1, **caractérisé en ce que** les deux conduits (38, 42, 138, 142) forment un angle approximativement de 90°.

3. Dispositif selon l'une quelconque des revendications précédentes, **caractérisé en ce que** le premier orifice (40, 140) est délimité par un bord périphérique disposé dans un plan contenant l'axe (48, 148) du second conduit (42,142).

4. Dispositif selon l'une quelconque des revendications précédentes, **caractérisé en ce que** la section du conduit (38, 138) au niveau du premier orifice (40, 140) est inférieure à celle du conduit (42, 142) au niveau du second orifice (44,144).

5. Dispositif selon la revendication 4, **caractérisé en ce que** le second conduit (142) comprend une réduction de diamètre.

6. Dispositif selon l'une quelconque des revendications précédentes, **caractérisé en ce qu'**il comprend un réservoir (58, 122) permettant de collecter les sécrétions nasales aspirées dans lequel découchent les conduits (38, 42, 138, 142).

7. Dispositif selon la revendication 6, **caractérisé en ce que** le réservoir (122) comprend un profil courbe (124) en face du conduit (142) de manière à ce que le flux d'air effectue un demi-tour afin de séparer les sécrétions du flux d'air.

8. Dispositif selon la revendication 6 ou 7, **caractérisé en ce que** le réservoir (122) est délimité par deux demi-coques (100, 102) assemblées selon un plan de jonction contenant les axes (146, 148) des deux conduits (138, 142).

9. Dispositif selon la revendication 8, **caractérisé en ce que** les deux conduits (138, 142) sont solidaires d'une demi-coque (100) et sont réalisés d'un seul tenant avec cette demi-coque (100).

10. Dispositif selon la revendication 9, **caractérisé en ce que** les surfaces extérieures des deux conduits (138, 142) comprennent des formes en saillie (114) qui coopèrent avec des formes en creux (116) prévues au niveau d'une demi-coque.

11. Dispositif selon l'une quelconque des revendications 8 à 10, **caractérisé en ce que** les demi-coques (100, 102) comprennent au moins une cloison (150) permettant de réduire le volume du réservoir (122).

12. Dispositif selon l'une quelconque des revendications 8 à 11, **caractérisé en ce qu'**une demi-coque (100) comprend au niveau de son bord périphérique au moins une nervure (152) qui se loge dans au moins une gorge (154) prévue au niveau du bord périphérique de l'autre demi-coque (102).

## Patentansprüche

1. Vorrichtung zum Absaugen von Nasensekreten mit einem Nasenstück (32, 132), das eine Öffnung (34, 134) aufweist, über die die Nasensekrete angesaugt werden, mit einer ersten Leitung (38, 138), deren erstes Ende mit dem Nasenstück (32, 132) verbunden ist und dessen anderes Ende in eine erste Öffnung (40, 140) mündet, und mit einer zweiten Leitung (42, 142), die ein Ende aufweist, das in eine zweite Öffnung (44, 144) mündet, **dadurch gekennzeichnet, dass** die beiden Leitungen (38, 42, 138, 142) einen Winkel kleiner gleich 90° bilden und dass die mit dem Nasenstück verbundene Öffnung (40, 140) in der Verlängerung der zweiten Leitung (42, 142) im Luftstrom angeordnet ist, der aus der Öffnung (44, 144) der zweiten Leitung (42, 142) herausströmt.

2. Vorrichtung nach Anspruch 1, **dadurch gekennzeichnet, dass** die beiden Leitungen (38, 42, 138, 142) einen Winkel von näherungsweise 90° bilden.

3. Vorrichtung nach einem der vorhergehenden Ansprüche, **dadurch gekennzeichnet, dass** die erste Öffnung (40, 140) von einem umlaufenden Rand begrenzt ist, der in einer Ebene angeordnet ist, die die Achse (48, 148) der zweiten Leitung (42, 142) enthält.

4. Vorrichtung nach einem der vorhergehenden Ansprüche, **dadurch gekennzeichnet, dass** der Querschnitt der Leitung (38, 138) im Bereich der ersten Öffnung (40, 140) kleiner als der Querschnitt der Leitung (42, 142) im Bereich der zweiten Öffnung (44, 144) ist.

5. Vorrichtung nach Anspruch 4, **dadurch gekennzeichnet, dass** die zweite Leitung (142) eine Verringerung des Durchmessers aufweist.

6. Vorrichtung nach einem der vorhergehenden Ansprüche, **dadurch gekennzeichnet, dass** diese einen Behälter (58, 122) aufweist, der es gestattet, die abgesaugten Nasensekrete zu sammeln, und in den die Leitungen (38, 42, 138, 142) münden.

7. Vorrichtung nach Anspruch 6, **dadurch gekennzeichnet, dass** der Behälter (122) gegenüber der Leitung (142) ein gekrümmtes Profil (124) aufweist, so dass der Luftstrom eine Kehrtwende ausführt, um die Sekrete vom Luftstrom zu trennen.

8. Vorrichtung nach Anspruch 6 oder 7, **dadurch gekennzeichnet, dass** der Behälter (122) von zwei Halbschalen (100, 102) begrenzt ist, die entlang einer Verbindungsebene zusammengefügt sind, die die Achsen (146, 148) der beiden Leitungen (138, 142) enthält.

9. Vorrichtung nach Anspruch 8, **dadurch gekennzeichnet, dass** die beiden Leitungen (138, 142) mit einer Halbschale (100) verbunden sind und mit dieser Halbschale (100) einstückig ausgeführt sind.

10. Vorrichtung nach Anspruch 9, **dadurch gekennzeichnet, dass** die äußeren Oberflächen der beiden Leitungen (138, 142) vorspringende Formen (114) aufweisen, die mit Hohlformen (116) zusammenwirken, die im Bereich einer Halbschale vorgesehen sind.

11. Vorrichtung nach einem der Ansprüche 8 bis 10, **dadurch gekennzeichnet, dass** die Halbschalen (100, 102) wenigstens eine Trennwand (150) aufweisen, die es gestattet, das Volumen des Behälters (122) zu verringern.

12. Vorrichtung nach einem der Ansprüche 8 bis 11, **dadurch gekennzeichnet, dass** eine Halbschale (100) im Bereich ihres äußeren Randes wenigstens einen Wulst (152) aufweist, der in wenigstens einer Vertiefung (154) zu liegen kommt, die im Bereich des äußeren Randes der anderen Halbschale (102) vorgesehen ist.

## Claims

1. Device making it possible to suction nasal secretions comprising a nasal tip (32, 132) with an opening (34, 134) via which nasal secretions are suctioned, a first pipe (38, 138) of which a first end is connected to the nasal tip (32, 132), with the other end emptying via a first opening (40, 140), and a second pipe (42, 142) with an end emptying via a second opening (44, 144), **characterized in that** the two pipes (38, 42, 138, 142) form an angle that is less than or equal to 90°, and the opening (40, 140) connected to the nasal tip is placed in the extension of the second pipe (42, 142), in the air stream exiting from the opening (44, 144) of the second pipe (42, 142).

2. Device according to Claim 1, wherein the two pipes (38, 42, 138, 142) form an approximately 90° angle.

3. Device according to any of the preceding claims, wherein the first opening (40, 140) is delimited by a peripheral edge placed in a plane that contains the axis (48, 148) of the second pipe (42, 142).

4. Device according to any of the preceding claims, wherein the cross-section of the pipe (38, 138) at the first opening (40, 140) is smaller than that of the pipe (42, 142) at the second opening (44, 144).

5. Device according to Claim 4, wherein the second pipe (142) comprises a reduction in diameter.

6. Device according to any of the preceding claims, wherein it comprises a reservoir (58, 122) making it possible to collect the suctioned nasal secretions into which the pipes (38, 42, 138, 142) are directed.

7. Device according to Claim 6, wherein the reservoir (122) comprises a curved profile (124) facing the pipe (142) in such a way that the air flow carries out a half-turn so as to separate the secretions from the air flow.

8. Device according to Claim 6 or 7, wherein the reservoir (122) is delimited by two half-shells (100, 102) assembled along a junction plane containing the axes (146, 148) of the two pipes (138, 142).

9. Device according to Claim 8, wherein the two pipes (138, 142) are integral with a half-shell (100) and are made in a single piece with this half-shell (100).

10. Device according to Claim 9, wherein the outside surfaces of two pipes (138, 142) comprise projecting shapes (114) that work with hollow shapes (116) provided at a half-shell.

11. Device according to any one of Claims 8 to 10, wherein the half-shells (100, 102) comprise at least one partition (150) making it possible to reduce the volume of the reservoir (122).

12. Device according to any one of Claims 8 to 11, wherein at its peripheral edge, a half-shell (100) comprises at least one rib (152) that is housed in at least one groove (154) provided at the peripheral edge of the other half-shell (102).
